# EUROPEAN PATENT SPECIFICATION

(11) **EP 1 424 987 B1**
(45) Date of publication and mention of the grant of the patent: **12.04.2006**
(21) Application number: 02772261.0
(22) Date of filing: 30.08.2002
(51) Int. Cl.: A61K 8/02, A61K 8/04, A61K 8/19, A61K 8/25, A61K 8/365, A61K 8/37, A61Q 19/10

(54) **COSMETIC EFFERVESCENT CLEANSING PILLOW**
KOSMETISCHES KISSEN MIT REINIGENDEM BRAUSEMITTEL
COUSSIN NETTOYANT EFFERVESCENT COSMETIQUE

(30) Priority: 13.09.2001 US 952510
(43) Date of publication of application: 09.06.2004
(73) Proprietor: UNILEVER PLC, London EC4P 4BQ (GB); UNILEVER N.V., 3013 AL Rotterdam (NL)
(72) Inventor: BERGQUIST, Paul, Roland, Unilever H&P Care USA, Trumbull, CT 06611 (US); SLAVTCHEFF, Craig, Stephen, Unilever H&P Care USA, Trumbull, CT 06611 (US)
(74) Representative: Mulder, Cornelis Willem Reinier
(86) International application number: PCT/EP2002/009826
(87) International publication number: WO 2003/022230

(56) References cited:
- WO-A-01/56542
- GB-A- 1 245 242
- US-A- 6 063 390
- US-A1- 2002 037 255
- US-B1- 6 280 757

## Description

The invention concerns a cleansing article based upon a water-insoluble sachet with an effervescent foamable powder actuatable with water held within the sachet.

Cleansing products have traditionally been marketed in the form of bar soaps, shower gels and mousses, the lather being generated by mechanical and aerosol dispensers. Mechanical implements have been used by consumers to assist in lather formation and physical removal of dirt through scrubbing. Wash cloths have been the implement of choice throughout recent history.

New formats for cleansing hold great consumer appeal. For instance, surfactant and conditioner compositions have been layered into apertured wipes such as disclosed in U.S. Patent 6,280,757 B1 (McAtee et al.). Open-mesh sponges such as described in U.S. Patent 6,066,607 (Gordon et al.) have assisted in generating foam from shower gels thereby improving speed and quality of lathering.

U.S. Patent 5,980,931 (Fowler et al.) describes a substantially dry, disposable personal care article wherein a surfactant system is dried onto a water-insoluble non-woven or similar substrate. A second generation of dual layered towelette has been reported. See WO 00/42961 (Smith), WO 01/08542 (Cen et al.), WO 01/08640 (Smith et al.), WO 01/08641 (Lorenzi et al.), WO 01/08655 (Phipps et al.), WO 01/08656 (Lorenzi et al.), WO 01/08657 (Lorenzi et al.) and WO 01/08658 (Cawkwell et al.) all describing disposable personal cleansing towelettes with lathering surfactant on at least a two layer cloth with one layer being a high loft substrate. Consumers are expected to place the towelette under water to generate a surfactant lather.

Another approach is described in U.S. Patent 6,063,390 (Farrell et al.) which discloses wiping articles that include an effervescent, preferably powdered, cleanser composition held within a pouch of a sachet. Water contact causes the composition to effervesce. Several problems exist with the aforedescribed "pillow" system.

Among those problems are maintenance of the powdered composition within the pouch (avoidance of seepage). Loss of powder is a particular problem where pouch walls are constructed of a high loft substrate. Other challenges involve control of gas generation to maintain a constant billowed pouch profile. Also it has been found difficult to heat-seal together substrates formed of different construction and types of materials.

A related technology is found in WO 01/56542 (Slavtcheff et al.) which describes a wiping article having an effervescent cleanser composition held within a sachet, at least one wall of the sachet including at least one pleat. The pleats are present to allow for expansion room to accommodate effervescent action within the sachet.

Accordingly, it is an advantage of the present invention to be able to provide an effervescent powdered composition containing sachet having an improved effectiveness at retaining powdered particles within a pouch of the sachet.

Another advantage of the present invention is to be able to provide an effervescent composition containing sachet wherein walls of the sachet allow for a controlled billowing of lather.

According to a first aspect there is provided an article for cleansing body surfaces, the article including:
an effervescent cleansing composition capable of generating a foam upon contact with water; and
a pouch comprising first and second water insoluble substrates, at least one being water permeable, the first and second water-insoluble substrates forming therebetween an area housing the cleansing composition, the first substrate comprising a spun lace or a carded/chemically bonded non-woven material and the second substrate comprising a high loft sheet bonded to a non-woven sheet selected from the group consisting of meltblown, spunbond and sheet combinations thereof.

A high loft water insoluble sheet forms at least part of the second substrate with a high loft surface on an outward side of the second substrate. The first substrate is of a different construction than that of the second. More particularly, the first substrate is a spun lace (hydroentangled) non-woven fabric or a carded/chemically bonded non-woven.

Advantages of the meltblown, spunbond and/or combination second substrate is that this material ic less adversely affected in its sealability when contaminent effervescent particles of the composition accidentally enter machinery during the pouch manufacture process. Moreover, meltblown and spunbond non-wovens allow for better sealability when sandwiched between the first substrate and any high loft sheet forming part of the second substate . The high loft sheet imparts to the second substrate increased aeration and improved latherability properties. In pouches where a high loft side is preferred, it is necessary to deploy the meltblown and/or spunbond substrate for the additional purpose of preventing powder from easily shaking free through the high loft sheet and exiting the pouch.

Further features and advantages of the present invention will become more readily apparent from consideration of the following drawing in which:
Fig. 1 is a perspective view of a pouch according to the present invention; and
Fig. 2 is a cross-sectional view taken along line 2-2 of Fig. 1.

Now it has been found that foam generating pouches based on effervescent water-activatable powders are best constructed with at least one wall being a sheet of a meltblown, spunbond and/or layered combination thereof substrate.

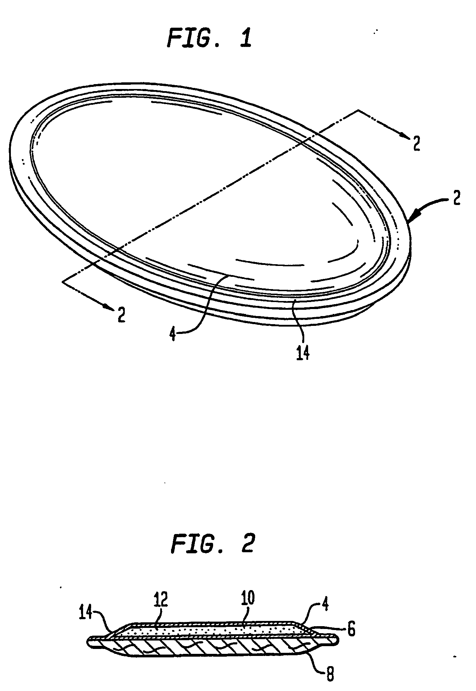

Fig. 1 illustrates pouch 2 of the present invention. The pouch has walls formed by a first spun lace substrate 4 and a second meltblown composite substrate 6, the latter being bonded to a high loft sheet 8. Chamber 10 formed between the first and second substrates 4, 6 serves to confine a powdered effervescent composition 12. Sheets 4 and 6 are ultrasonically sealed along a circumferential track 14 to insure no loss of the powdered composition.

Copious foam is generated when pouch 2 is wetted with water, much in the same manner as a toilet bar is lathered. Physically, the pouch is constructed in an oval shape with a size fittable within a human hand thereby simulating a toilet bar. Cross-sectional diameters may range from about 2 to about 15 cm, preferably from about 5 to about 10 cm.

Sheet 6 may be a single meltblown layer but preferably it is a combination of several layers. One embodiment utilizes the three layers of spunbond/meltblown/spunbond (SMS) and another utilizes four layers representing spunbond/meltblown/meltblown/spunbond (SMMS) construction. In these systems the meltblown fibers are very compact and act as a barrier against loss of powder. The spunbond layers are provided for strength and softness.

The meltblown web can be any meltblown web made from a thermoplastic polymer having a melting point greater than about 50°C. A preferred polymer is polypropylene, which is the most commonly used polymer for making meltblown webs. Other suitable polymers include poly(butylene terephthalate), polycaprolactam, poly(ethylene terephthalate) and polyethylene.

The process for making meltblown webs is well known in the art and is used extensively for manufacturing a wide variety of commercial nonwoven products. Representative examples of the meltblowing process are described in U.S. Pat. No. 3,978,185 to Buntin et al. dated August 31, 1976; U.S. Patent No. 4,298,649 to Meitner dated November 3, 1981; and U.S. Patent No. 4,100,324 to Anderson et al. dated July 11, 1978, all herein incorporated by reference. It will be appreciated, however, that other meltblowing processes will produce webs suitable for purposes of this invention. The meltblown web can be combined or laminated to other supporting webs, such as spunbonded webs, in order to impart strength or other attributes to the product.

The basis weight for a single sheet of the meltblown base webs of this invention can range from about 20 to about 300 grams per square meter. Preferably the basis weight will be from about 80 to about 250, and most preferably from about 100 to about 200 grams per square meter.

Spunbonding entails extruding a multiplicity of continuous thermoplastic polymer strands through a multiplicity of die orifices in a downward direction onto a moving surface where the extruded strands are collected in randomly distributed fashion. The randomly deposited strands are then bonded together in a heated nip to provide sufficient integrity to the resulting nonwoven web of continuous fibers. Spunbonded webs are characterized by a high strength/weight ratio, isotropic strength, high porosity, and good abrasion resistance.

Meltblowing differs from spunbonding in that the extruded polymer strands are broken up and dispersed into individual fibers by a forced air stream before being deposited onto the collecting surface. In addition, the fibers are substantially cooled by the air so that they do not significantly crystallize and/or bond together. Bonding of the web to retain integrity and strength occurs as a separate downstream operation.

A most preferred material for the second substrate is a non-woven meltblown/spunbond substrate available from the Polyprop Corporation.

The first substrate preferably is either a spun lace or a carded/chemically bonded non-woven water-insoluble material.

Particularly preferred is a cloth NC008 (Image Spun Lace) available from the PGI Corporation.

When present, the high loft sheet is generally fluid-permeable. As used herein, "high loft" means that the sheet has a density of from about 0.00005 g/cm³ to about 0.1 g/cm³, preferably from about 0.001 g/cm³ to about 0.09 g/cm³ and a thickness of from about 0.1 cm to about 5 cm.

As used herein, "nonwoven" means that the layer does not comprise fibers which are woven into a fabric but the layer need not comprise fibers at all, e.g., formed films, sponges, foams, scrims, etc. When the layer comprises fiber, the fibers can either be random (i.e. randomly aligned) or they can be carded (i.e. combed to be oriented in primarily one direction).

For purposes of this invention, the most preferred high loft material is a needle punched composite sold by Union Wadding Corporation.

A first component.of compositions 12 within the pouch is that of an acidic material. Suitable for this purpose are any acids, and preferably those present in dry solid form. Especially appropriate are C₂-C₂₀ organic mono- and polycarboxylic acids and especially alpha- and beta-hydroxycarboxylic acids; C₂-C₂₀ organophosphorus acids such as phytic acid; C₂-C₂₀ organosulfur acids such as toluene sulfonic acid; and peroxides such as hydrogen peroxide. Typical hydroxycarboxylic acids include adipic, glutaric, succinic, tartaric, malic, maleic, lactic, salicylic and citric acids as well as acid forming lactones such as gluconolactone and glucarolactone. Most preferred is citric acid. Also suitable as acid material may be encapsulated acids. Typical encapsulating material may include water soluble synthetic or natural polymers such as polyacrylates (e.g. encapsulating polyacrylic acid), cellulosic gums, polyurethane and polyoxyalkylene polymers. By the term "acid" is meant any substance which when dissolved in deionized water at 1% concentration will have a pH of less than 7, preferably less than 6.5, optimally less than 5. These acids preferably at 25°C are in solid form, i.e. having melting points no less than 25°C. Concentrations of the acid should range from about 0.5 to about 80%, preferably from about 10 to about 65%, optimally from about 20 to about 45% by weight of the total composition.

A second component of compositions within the pouch is that of an alkaline material. The alkaline material is a substance which can generate a gas such as carbon dioxide, nitrogen or oxygen, i.e. effervesce, when contacted with water and the acidic material. Suitable alkaline materials are anhydrous salts of carbonates and bicarbonates, alkaline peroxides (e.g. sodium perborate and sodium percarbonate) and azides (e.g. sodium azide). Preferably the alkaline material is sodium or potassium bicarbonate. Amounts of the alkaline material may range from about 1 to about 80%, preferably from about 5 to about 49%, more preferably from about 15 to about 40%, optimally from about 20 to about 35% by weight of the total composition.

By the term "anhydrous" is meant the presence of no more than about 15%, preferably no more than about 5% and optimally no more than 1% water by weight of the total composition. Water of hydration is not considered to be water for purposes of the anhydrous definition. However, it is preferred to minimize, preferably to eliminate any water of hydration.

Advantageously the combined amount of acidic and alkaline materials will be at least about 1.5%, preferably from about 40 to about 95%, optimally from about 60 to about 80% by weight of the total composition.

An optimal further component of compositions according to the present invention is that of a surfactant, preferably a dry surfactant solid at 20°C. Most suitable for the present invention is sodium cocoyl isethionate. Other useful surfactants include sodium methyl cocoyl taurate, sodium lauroyl sarcosinate and sodium lauryl sulfate. Surfactants may be of the anionic, cationic, nonionic, amphoteric, zwitterionic varieties and combinations thereof. Amounts of the surfactant may range from about 0.1 to about 30%, preferably from about 1 to about 30%, optimally from about 8 to about 20% by weight of the total composition.

A variety of skin benefit agents may be included to improve afterfeel properties. Advantageously these substances will be available as anhydrous dry powders. Alternatively these substances may be liquids deposited upon or into a powdered substrate (e.g. calcium silicate or zeolite) to achieve a resultant dry flowing powder. Within the skin benefit agent scope are several categories of materials. These include emollients, antiaging actives, antibacterials and fungicides, skin lighteners, sunscreens and combinations thereof. Amounts of the skin benefit agents may range from about 0.001 to about 40%, preferably from about 0.1 to about 20%, more preferably from about 0.5 to about 10%, optimally between about 1 and about 5% by weight of the total composition.

Emollients may be in the form of natural or synthetic esters, silicone oils, hydrocarbons, starches, fatty acids and mixtures thereof. Typically the emollient may range in concentration from about 0.1 to about 35% by weight of the total composition.

Silicone oils may be divided into the volatile and nonvolatile variety, The term "volatile" as used herein refers to those materials which have a measurable vapor pressure at ambient temperature. Volatile silicone oils are preferably chosen from cyclic or linear polydimethylsiloxanes containing from 3 to 9, preferably from 4 to 5, silicon atoms.

Linear volatile silicone materials generally have viscosities less than about 5 centistokes at 25°C while cyclic materials typically have viscosities of less than about 10 centistokes.

Nonvolatile silicone oils useful as an emollient material include polyalkyl siloxanes, polyalkylaryl siloxanes and polyether siloxane copolymers. The essentially non-volatile polyalkyl siloxanes useful herein include, for example, polydimethyl siloxanes with viscosities of from about 5 to about 100,000 centistokes at 25°C. Among the preferred non-volatile emollients useful in the present compositions are the polydimethyl siloxanes having viscosities from about 10 to about 400 centistokes at 25°C.

Among the ester emollients are:
(1) Alkenyl or alkyl esters of fatty acids having 10 to 22 carbon atoms. Examples thereof include isoarachidyl neopentanoate, isononyl isonanonoate, oleyl myristate, oleyl stearate, and oleyl oleate.
(2) Ether-esters such as fatty acid esters of ethoxylated fatty alcohols.
(3) Polyhydric alcohol esters. Ethylene glycol mono and di-fatty acid ester, diethylene glycol mono- and di-fatty acid esters, polyethylene glycol (200-8000) mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters.
(4) Wax esters such as beeswax, spermaceti, myristyl myristate, stearyl stearate and arachidyl behenate.
(5) Sterols esters, of which cholesterol fatty acid esters are examples thereof.
(6) Triglycerides such as sunflower seed oil, maleated sunflower seed oil, polycottonseedate, borage seed oil and safflower oil.

Hydrocarbons suitable as emollients include petrolatum, mineral oil, isoparaffins and hydrocarbon waxes such as polyethylene.

Starches are also suitable emollients. Typical of this class is tapioca and arabinogalactan.

Fatty acids may also be suitable as emollients. The fatty acids normally have from 10 to 30 carbon atoms. Illustrative of this category are pelargonic, lauric, myristic, palmitic, stearic, isostearic, hydroxystearic, oleic, linoleic, ricinoleic, arachidic, behenic and erucic acids.

Antiaging actives are also useful as skin benefit agents. Included within this category are vitamins, retinoids and combinations thereof. Amounts of these materials may range from about 0.001 to about 20% by weight of the total composition. Suitable vitamins include ascorbic acid, Vitamin B₆, Vitamin B₁₂, tocopherol as well as salts and C₁₋C₂₀ esters thereof. Suitable retinoids include retinoic acid as well as its C₁-C₂₂ esters and salts, retinol and C₁₋C₂₂ fatty esters of retinol including retinyl linoleate.

Another class of antiaging actives are the alpha- and beta-hydroxycarboxylic acids and salts thereof. Representative of this group are glycolic acid, lactic acid, malic acid, hydroxyoctanoic acid, salicylic acid and mixtures of these as well as their salts. Suitable salts are the alkalimetal, ammonium and C₁-C₁₀ alkanol ammonium salts.

Antibacterials and fungicidals may also be included as skin benefit agents. Representative of these categories are triclosan, tricloban, hexetidene, chlorhexadene, gluconates, zinc salts (e.g. zinc citrate and zinc phenolsulfonate) and combinations thereof.

Skin lighteners may also be included under the skin benefit agents. Typical of this category are niacinamide, kojic acid, arbutin, vanillin, ferulic acid and esters thereof, resorcinol, hydroquinone, placental extract and combinations thereof.

Sunscreens may also be included as skin benefit agents. Particularly preferred are such materials as ethylhexyl p-methoxycinnamate, available as Parsol® MCX, avobenzene available as Parsol® 1789 and benzophenone-3, also known as Oxybenzone. Inorganic sunscreen actives may be employed such as microfine titanium dioxide, polyethylene and various other polymers. Amounts of the sunscreen agents may generally range from 0.1 to 30%, preferably from 2 to 20%, optimally from 4 to 10% by weight.

Adjunct functional agents may also be incorporated into compositions of the present invention. These include electrolytes, thickeners and mixtures thereof. Amounts of these substances may range from about 0.1 to about 20%, preferably from about 0.3 to about 10%, optimally between about 0.5 and about 5% by weight of the total composition.

Electrolytes may be selected from alkali, alkaline earth or ammonium salts of phosphates, silicates, halides, sulphates and mixtures thereof. Typical phosphates are potassium polymetaphosphate, sodium tripolyphosphate, sodium tetrapyrophosphate, sodium or potassium pyrophosphate and sodium hexametaphosphate. Most preferred is potassium polymetaphosphate available as Lipothix 100B® which is a 70:30 mixture of potassium polymetaphosphate and sodium bicarbonate, available from Lipo Chemicals, Inc., Paterson, New Jersey. Preferred sulphates are the magnesium sulphates.

Thickeners which may improve afterfeel properties on skin include inorganic or organic substances. A particularly preferred inorganic thickener is sodium magnesium silicate commercially available as Optigel SH®. Organic thickeners include alginic acid as well as sodium and calcium alginates, sodium carboxymethyl cellulose, hydroxypropyl methylcellulose, hydroxypropyl cellulose, hydroxyethyl cellulose and combinations thereof. Most preferred is alginic acid commercially available as Kelacid® from Sud-Chemie Rheologicals, Louisville, Kentucky. Alginic acid is highly effective at removing the slimy feel associated with deposits of alkaline material which are not fully rinsed away from the skin. Amounts of the thickener may range from about 0.1 to about 20%.

Polysaccharides useful in this invention are dry solid anhydrous substances such as sorbitol, sugars, (such as trehalose), starches, modified starches (e.g. aluminum octenyl succinate) and mixtures thereof. Most preferred is sorbitol.

Deposition aids may also be incorporated in compositions of the present invention. These assist in depositing skin benefit agents onto the skin surface. Particularly effective are cationic monomers and polymers for this purpose. Illustrative are the following: Lauryltrimethylammonium chloride; Stearyltri(2-hydroxyethyl) ammonium chloride; Lauryldimethylbenzylammonium chloride; Cetyldimethylbenzylammonium chloride; Cetylpyridinium chloride; Polydiallyldimethylammonium chloride; Diallydimethylammonium salt copolymerized with acrylamide; Guar hydroxypropyltrimonium chloride; Cationic hydroxyethylcellulosics; Cationic hydroxyethylcellulosics; Cetyltrimethylammonium chloride; Decyldimethyloctylammonium chloride; and Myristyltrimethylammonium chloride.

Most preferred for purposes of this invention are cationic guar gums such as Jaguar C13S® which is guar hydroxypropyltrimonium chloride and Polyquaternium 7 commercially available as Merquat 2200. Amounts of the deposition aid may range from about 0.01 to about 1%, preferably from about 0.05 to about 0.5%, optimally from about 0.1 to about 0.3% by weight.

Advantageously an emotive agent such as a fragrance and/or botanical extract are included with the effervescent cleansing composition. Fragrances and botanicals are often liquids. For this reason it may be necessary to uniformly distribute and allow absorption of liquid components into the solid powder. One method of best achieving this is to spray these liquids onto the solids. Amounts of the fragrance and/or botanicals combined may be at levels from about 0.1 to about 3%, preferably from 0.5 to 2%, optimally from 0.8 to 1.5% by weight of the total composition.

The term "fragrance" is defined as a mixture of odoriferous components, optionally mixed with a suitable solvent diluent or carrier, which is employed to impart a desired odor. Particular preferred odoriferous components are cyclic and acyclic terpenes and terpenoids. These materials are based upon isoprene repeating units. Examples include alpha and beta pinene, myrcene, geranyl alcohol and acetate, camphene, dl-limonene, alpha and beta phellandrene, tricyclene, terpinolene, allocimmane, geraniol, nerol, linanool, dihydrolinanool, citral, ionone, methyl ionone, citronellol, citronellal, alpha terpineol, beta terpineol, alpha fenchol, borneol, isoborneol, camphor, terpinen-1-ol, terpin-4-ol, dihydroterpineol, methyl chavicol, anethole, 1,4 and 1,8 cineole, geranyl nitrile, isobornyl acetate, linalyl acetate, caryophyllene, alpha cedrene, guaiol, patchouli alcohol, alpha and beta santalol and mixtures thereof. Botanicals of particular use in the present invention include yarrow, chamomile, jasmine, lavender, horse chestnut, sage, thyme, yucca, coltsfoot and mixtures thereof.

Preservatives can desirably be incorporated into the cosmetic compositions of this invention to protect against the growth of potentially harmful microorganisms. Suitable traditional preservatives for compositions of this invention are alkyl esters of para-hydroxybenzoic acids. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are phenoxyethanol, methyl paraben, propyl paraben, imidazolidinyl urea, sodium dehydroacetate and benzyl alcohol. Preservatives are preferably employed in amounts ranging from 0.01% to 2% by weight of the composition.

Colorants may also be included in compositions of the present invention. These substances may range from about 0.05 to about 5%, preferably between 0.1 and 3% by weight.

It is important that the substrate sheets are not readily torn open through consumer rubbing of the article. Unlike laundry sachet articles, pouches of the present invention should not rupture to allow dispersion of their granular contents into wash water. Rather it is intended for all composition components to exit by dissolution through the permeable walls of the pouch.

Skin surfaces against which articles of the present invention are useful include face, body, scalp, axilla and even legs/feet. When the article is a foot cleanser, it would be advantageous for the pouch on one of its sides to be coarse while the second of the substrates may be soft and gentle.

Articles according to the present invention may be formed in the following manner. Constituents of the effervescent cleansing composition are placed into a dry mill or similar apparatus and blended until a uniformally distributed powder results. Thereafter, fragrance/botanical component as a Phase B is sprayed into the dry mill with concurrent agitation of the powdered composition.

Rolls of first and second substrate sheets are unwound from different sides of a charging position. The effervescent cleansing composition is placed into a hopper positioned over the charging position and between the substrate sheets. A discrete charge of powdered composition is released directed between the sheets and caught in a partially formed pillow. At this point all edges defining the pouch are sealed in register trapping the effervescent cleansing composition within. Cutters then separate one sealed section from another thereby forming the article. One or more of the articles are then packaged within a moisture impermeable outer package such as a laminated foil bag to prevent activation of the effervescent system during storage.

Ultrasonic welding may be employed as an alternative to heat-sealing of the first and second substrates together. Thread stitching, glue application or other closure mechanisms may also be utilized.

Except in the operating and comparative examples, or where otherwise explicitly indicated, all numbers in this description indicating amounts of material are to be understood as modified by the word "about".

The term "comprising" is meant not to be limiting to any subsequently stated elements but rather to encompass non-specified elements of major or minor functional importance. In other words the listed steps, elements or options need not be exhaustive.

The following examples will more fully illustrate embodiments of this invention. All parts, percentages and proportions referred to herein and in the appended claims are by weight unless otherwise indicated.

### EXAMPLE 1 (comparative)

An effervescent cleansing composition was prepared according to the formulation reported in Table I. Phase A was dry blended in a high speed shearing mixer. Fragrance was then sprayed onto the resultant powder as a Phase B. Three grams of the resultant powder are then placed into a pocket of a 5 cm by 8 cm pouch. Walls of the pouch are formed by heat sealing the circumferences of a non-woven spun lace web to a non-woven SMMS web.

**TABLE I**

| **INGREDIENT** | **WEIGHT %** |
|---|---|
| **PHASE A** | |
| Sodium Bicarbonate | 34.5 |
| Citric Acid (Anhydrous) | 40.4 |
| Sodium Cocoyl Isethionate (Powder) | 11.6 |
| Sodium Sesquicarbonate | 5.0 |
| Lipothix 100B® (Potassium Polymetaphosphate/Bicarbonate 70:30) | 0.5 |
| Optigel SH® (Sodium Magnesium Silicate) | 1.0 |
| Kelacid® (Alginic Acid) | 1.0 |
| Sorbitol | 5.0 |

| **PHASE B** | |
|---|---|
| Fragrance | 1.0 |

### EXAMPLE 2 (comparative)

Another effervescent cleansing composition was prepared according to the formulation reported in Table II. The composition is then sealed into a pouch as described in Example 1.

**TABLE II**

| **INGREDIENT** | **WEIGHT %** |
|---|---|
| **PHASE A** | |
| Sodium Bicarbonate | 32.3 |
| Citric Acid (Anhydrous) . | 41.1 |
| Sodium Cocyl Isethionate (Powder) | 11.6 |
| Sodium Sesquicarbonate | 5.0 |
| Lipothix 100B® (Potassium Polymetaphosphate/Bicarbonate 70:30) | 0.5 |
| Optigel SH® (Sodium Magnesium Silicate) | 1.0 |
| Kelacid® (Alginic Acid) | 1.0 |
| Sorbitol | 5.0 |
| Laracare A200® (Arabinogalactan) | 1.0 |
| Ascorbic Acid | 0.5 |

| **PHASE B** | |
|---|---|
| Fragrance | 1.0 |

### EXAMPLE 3 (comparative)

A face cleansing effervescent composition was prepared according to the formulation reported in Table III. The composition is then sealed into a pouch as described in Example 1.

**TABLE III**

| **INGREDIENT** | **WEIGHT %** |
|---|---|
| **PHASE A** | |
| Sodium Bicarbonate | 33.6 |
| Citric Acid (Anhydrous) | 39.0 |
| Sodium Cocyl Isethionate (Powder) | 3.0 |
| Sodium Methyl Cocoyl Taurate | 6.0 |
| Sodium Lauryl Sulfate | 2.5 |
| Sodium Sesquicarbonate | 5.0 |
| Lipothix 100B® (Potassium Polymetaphosphate/Bicarbonate 70:30) | 0.5 |
| Optigel SH® (Sodium Magnesium Silicate) | 2.0 |
| Tapioca | 5.5 |
| Methyl Gluceth 20-Benzoate | 2.0 |
| Guar Hydroxypropyl Trimonium Chloride | 0.25 |

| **PHASE B** | |
|---|---|
| Fragrance | 0.65 |

### EXAMPLE 4

A still further effervescent cleansing composition according to the present invention may be prepared according to the formulation reported under Table IV. The dry mixed ingredients at a weight level of 3.5 grams per sample are placed into an oval pouch of major and minor elliptical axis of 9 cm and 5.5 cm length. The powdered sample is placed between a layer of spunlace substrate and an SMS layer to which a high loft sheet is sealed. All sides of the pouch are welded by ultrasonic heat to ensure against powder escaping.

**TABLE IV**

| **INGREDIENT** | **WEIGHT %** |
|---|---|
| Anhydrous Citric Acid | 24.00 |
| Sodium Bicarbonate | 24.00 |
| Sodium Lauryl Sulfoacetate | 3.75 |
| Sodium Cocoyl isethionate | 3.75 |
| Polyquaternium 7 | 0.50 |
| Sodium C14-C16 Olefin Sulfonate | 3.75 |
| Guar hydroxypropyltrimonium Chloride | 0.40 |
| Ascorbyl Palmitate | 0.01 |
| Isocetyl Behenate | 5.00 |
| Sodium Stearoyl Lactylate | 3.00 |
| Disodium Dimethicone Copolyol Sulfosuccinate | 1.00 |
| Vitamin A Palmitate | 0.01 |
| Sodium Lauroyl Lactylate | 5.00 |
| PEG 8000 | 5.00 |
| Vitamin E | 0.40 |
| Calcium Silicate | 9.00 |
| Maltodextrin | 11.13 |
| Fragrance | 0.30 |

## Claims

1. An article for cleansing body surfaces, the article comprising:
an effervescent cleansing composition capable of generating a foam upon contact with water; and
a pouch comprising first and second water-insoluble substrates, at least one being water permeable, the first and second substrates forming therebetween an area housing the cleansing composition, the first substrate comprising a spun lace or a carded/chemically bonded non-woven material and the second substrate comprising a high-loft sheet bonded to a non-woven sheet selected from the group consisting of meltblown, spunbond and sheet combinations thereof.

2. The article according to claim 1 wherein the cleansing composition is powdered.

3. The article according to claim 1 or claim 2 wherein the nonwoven sheet is selected from the group consisting of meltblown/spunbond, spunbond/meltblown/spunbond and spunbond/meltblown/meltblown/spunbond layered construction.

4. The article according to any of the preceding claims wherein the cleansing composition comprises from about 0.5 to about 80% by weight of an acid and from about 1 to about 80% by weight of an alkaline material.

5. The article according to claim 4 wherein the composition further comprises from about 0.1 to about 30% by weight of a surfactant.

6. The article according to claim 5 wherein the composition further comprises from about 0.001 to about 40% by weight of a skin benefit agent.

7. A method for cleansing body surfaces, the method comprising:
(i) preparing an article comprising:
an effervescent cleansing composition capable of generating a foam upon contact with water; and
a pouch comprising first and second water-insoluble substrates, at least one being water permeable, the first and second substrates forming therebetween an area housing the cleansing composition, the first substrate comprising a spun lace or a carded/chemically bonded non-woven material and the second substrate comprising a high loft sheet bonded to a non-woven sheet selected from the group consisting of meltblown, spunbond and sheet combinations thereof;
(ii) wetting the article to generate an effervescent cleansing foam; and
(iii) applying the wetted article with generated effervescent cleansing foam onto parts of a user's body surfaces and cleansing therewith those surfaces.

8. An article for cleansing body surfaces, the article comprising:
an effervescent cleansing powdered composition capable of generating a foam upon contact with water, the composition being anhydrous and comprising;
(i) from about 1 to about 80% of an alkaline material;
(ii) from about 0.5 to about 80% of an acid material; and
(iii)from about 0.1 to about 30% of a surfactant a pouch formed of first and second water-insoluble substrates, at least one being water permeable, the first and second substrates forming therebetween an area housing the cleansing powdered composition, the first substrate comprising a spun lace or a carded/chemically bonded non-woven material and the second substrate comprises a high loft sheet bonded to a non-woven sheet selected from the group consisting of meltblown, spunbond and sheet combinations thereof.

9. An article for cleansing body surfaces, the article comprising:
an effervescent cleansing composition capable of generating a foam upon contact with water; and
a pouch comprising first and second water-insoluble substrates, at least one being water permeable, the first and second substrates forming therebetween an area housing the cleansing composition, the second substrate comprising a high loft sheet bonded to a non-woven sheet selected from the group consisting of meitblown, spunbond and sheet combinations thereof and the first substrate comprising a spun lace sheet.

## Patentansprüche

1. Gegenstand zum Reinigen von Körperoberflächen, wobei der Gegenstand umfasst:
eine sprudelnde Reinigungszusammensetzung, die bei Kontakt mit Wasser einen Schaum erzeugen kann, und
einen Beutel, umfassend erste und zweite in Wasser unlösliche Substrate, wobei mindestens eines für Wasser permeabel ist, wobei die ersten und zweiten Substrate dazwischen einen Bereich bilden, der die Reinigungszusammensetzung aufnimmt, wobei das erste Substrat ein Spun-Lace- oder ein kardiertes/chemisch gebundenes Vliesmaterial umfasst, und das zweite Substrat ein High-Loft-Tuch, das an eine Vliesfolie, ausgewählt aus der Gruppe, bestehend aus schmelzgeblasenen, spun-bonded und Vlieskombinationen davon, gebunden ist, umfasst.

2. Gegenstand nach Anspruch 1, worin die Reinigungszusammensetzung pulverisiert ist.

3. Gegenstand nach Anspruch 1 oder Anspruch 2, worin die Vliesfolie ausgewählt ist aus der Gruppe, bestehend aus schmelzgeblasenem/spun-bonded, spun-bonded/schmelzgeblasenem/spun-bonded und spun-bonded/schmelzgeblasenem/schmelzgeblasenem/spun-bonded Schichtaufbau.

4. Gegenstand nach einem der vorangehenden Ansprüche, worin die Reinigungszusammensetzung etwa 0,5 bis etwa 80 Gew.-% einer Säure und etwa 1 bis etwa 80 Gew.-% eines alkalischen Materials umfasst.

5. Gegenstand nach Anspruch 4, worin die Zusammensetzung weiterhin etwa 0,1 bis etwa 30 Gew.-% eines Tensids umfasst.

6. Gegenstand nach Anspruch 5, worin die Zusammensetzung weiterhin etwa 0,001 bis etwa 40 Gew.-% eines Hautvorteilsmittels umfasst.

7. Verfahren zum Reinigen von Körperoberflächen, wobei das Verfahren umfasst:
(i) Herstellung eines Gegenstands, umfassend
eine sprudelnde Reinigungszusammensetzung, die bei Kontakt mit Wasser einen Schaum erzeugen kann, und einen Beutel, umfassend erste und zweite in Wasser unlösliche Substrate, wobei mindestens eines für Wasser permeabel ist, wobei die ersten und zweiten Substrate dazwischen einen Bereich bilden, der die Reinigungszusammensetzung aufnimmt, wobei das erste Substrat ein Spun-Lace- oder ein kardiertes/chemisch gebundenes Vliesmaterial umfasst, und das zweite Substrat ein High-Loft-Tuch, das an eine Vliesfolie, ausgewählt aus der Gruppe, bestehend aus schmelzgeblasenen, spun-bonded und Vlieskombinationen davon gebunden ist, umfasst,
(ii) Benetzen des Gegenstandes zur Erzeugung von sprudelndem Reinigungsschaum und
(iii) Auftragen des befeuchteten Gegenstands mit erzeugtem sprudelndem Reinigungsschaum auf Teile von Körperoberflächen des Anwenders und Reinigen von jenen Oberflächen damit.

8. Gegenstand zur Reinigung von Körperoberflächen, wobei der Gegenstand umfasst:
eine sprudelnde reinigende pulverisierte Zusammensetzung, die bei Kontakt mit Wasser einen Schaum erzeugen kann, wobei die Zusammensetzung wasserfrei ist und umfasst:
(i) etwa 1 bis etwa 80 % eines alkalischen Materials
(ii) etwa 0,5 bis etwa 80 % eines sauren Materials und
(iii) etwa 0,1 bis etwa 30 % eines Tensids
einen Beutel, gebildet aus ersten und zweiten in Wasser unlöslichen Substraten, wobei mindestens eines für Wasser permeabel ist, wobei die ersten und zweiten Substrate dazwischen einen Bereich bilden, der die pulverisierte Reinigungszusammensetzung aufnimmt, wobei das erste Substrat ein Spun-Lace- oder ein kardiertes/chemisch gebundenes Vliesmaterial umfasst, und das zweite Substrat ein High-Loft-Tuch, das an eine Vliesfolie, ausgewählt aus der Gruppe, bestehend aus schmelzgeblasenen, spun-bonded und Vlieskombinationen davon, gebunden ist, umfasst.

9. Gegenstand zum Reinigen von Körperoberflächen, wobei der Gegenstand umfasst:
eine sprudelnde Reinigungszusammensetzung, die bei Kontakt mit Wasser einen Schaum erzeugen kann, und
einen Beutel, umfassend erste und zweite in Wasser unlösliche Substrate, wobei mindestens eines für Wasser permeabel ist, wobei die ersten und zweiten Substrate dazwischen einen Bereich bilden, der die Reinigungszusammensetzung aufnimmt, wobei das zweite Substrat ein High-Loft-Tuch, das an eine Vliesfolie, ausgewählt aus der Gruppe, bestehend aus schmelzgeblasenen, spun-bonded und Vlieskombinationen davon, gebunden ist, umfasst, und das erste Substrat ein Spun-Lace-Tuch umfasst.

## Revendications

1. Article destiné au nettoyage des surfaces corporelles, l'article comprenant :
une composition nettoyante effervescente capable de générer de la mousse au contact de l'eau ; et
un sachet comprenant un premier et un second substrats insolubles dans l'eau, dont l'un au moins est perméable à l'eau, le premier et le second substrats ménageant entre eux un espace abritant la composition nettoyante, le premier substrat comprenant un matériau non tissé hydrolié ou cardé/chimiquement lié, et le second substrat comprenant une nappe à pouvoir gonflant élevé liée à une nappe non tissée choisie dans le groupe constitué des nappes meltblown, spunbond et des combinaisons en nappe de celles-ci.

2. Article selon la revendication 1, dans lequel la composition nettoyante se présente sous la forme d'une poudre.

3. Article selon les revendications 1 ou 2, dans lequel la nappe non tissée est choisie dans le groupe constitué des nappes multicouches meltblown/spunbond, spunbond/meltblown/spunbond et spunbond/meltblown/ meltblown/spunbond.

4. Article selon l'une quelconque des revendications précédentes, dans lequel la composition nettoyante comprend entre environ 0,5 et environ 80 % en poids d'un matériau acide et entre environ 1 et environ 80 % en poids d'un matériau alcalin.

5. Article selon la revendication 4, dans lequel la composition comprend, en outre, entre environ 0,1 et environ 30 % en poids d'un tensioactif.

6. Article selon la revendication 5, dans lequel la composition comprend, en outre, entre environ 0,001 et environ 40 % en poids d'un agent bénéfique pour la peau.

7. Procédé de nettoyage des surfaces corporelles , le procédé comprenant :
(i) la préparation d'un article comprenant :
une composition nettoyante effervescente capable de générer de la mousse au contact de l'eau ; et
un sachet comprenant un premier et un second substrats insolubles dans l'eau, dont l'un au moins est perméable à l'eau, le premier et le second substrats ménageant entre eux un espace abritant la composition nettoyante, le premier substrat comprenant un matériau non tissé hydrolié ou cardé/chimiquement lié et le second substrat comprenant une nappe à pouvoir gonflant élevé liée à une nappe non tissée choisie dans le groupe constitué des nappes meltblown, spunbond et des combinaisons en nappe de celles-ci ;
(ii) l'humidification de l'article afin de générer une mousse nettoyante effervescente ; et
(iii) l'application de l'article humidifié porteur de la mousse nettoyante effervescente ainsi produite sur certaines parties des surfaces corporelles d'un utilisateur et le nettoyage desdites surfaces avec ledit article.

8. Article destiné au nettoyage des surfaces corporelles, l'article comprenant :
une composition nettoyante effervescente en poudre capable de générer de la mousse au contact de l'eau, ladite composition étant anhydre et comprenant :
(i) entre environ 1 et environ 80 % d'un matériau alcalin ;
(ii) entre environ 0,5 et environ 80 % d'un matériau acide ; et
(iii) entre environ 0,1 et environ 30 % d'un tensioactif
un sachet constitué d'un premier et d'un second substrats insolubles dans l'eau, dont l'un au moins est perméable à l'eau, le premier et le second substrats ménageant entre eux un espace abritant la composition nettoyante en poudre, le premier substrat comprenant un matériau non tissé hydrolié ou cardé/chimiquement lié et le second substrat comprenant une nappe à pouvoir gonflant élevé liée à une nappe non tissée choisie dans le groupe constitué des nappes meltblown, spunbond et des combinaisons en nappe de celles-ci.

9. Article destiné au nettoyage des surfaces corporelles, l'article comprenant :
une composition nettoyante effervescente capable de générer de la mousse au contact de l'eau ; et
un sachet comprenant un premier et un second substrats insolubles dans l'eau, dont l'un au moins est perméable à l'eau, le premier et le second substrats ménageant entre eux un espace abritant la composition nettoyante, le second substrat comprenant une nappe à pouvoir gonflant élevé liée à une nappe non tissée choisie dans le groupe constitué des nappes meltblown, spunbond et des combinaisons en nappe de celles-ci et le premier substrat comprenant une nappe hydroliée.
